# EUROPÄISCHE PATENTSCHRIFT

(11) **EP 4 268 731 B1**
(45) Veröffentlichungstag und Bekanntmachung des Hinweises auf die Patenterteilung: **26.08.2026**
(21) Anmeldenummer: 23157931.9
(22) Anmeldetag: 22.02.2023
(51) Int. Cl.: A61B 10/00

(54) **VORRICHTUNG ZUM SAMMELN EINER STUHLPROBE EINES LEBEWESENS**
DEVICE FOR COLLECTING A STOOL SAMPLE OF A LIVING BEING
DISPOSITIF DE COLLECTE D'UN ÉCHANTILLON FÉCAL D'UN ÊTRE VIVANT

(30) Priorität: 29.04.2022 DE 202022102320 U
(43) Veröffentlichungstag der Anmeldung: 01.11.2023
(73) Patentinhaber: Labor LS SE & Co. KG, 97708 Bad Bocklet-Großenbrach (DE)
(72) Erfinder: BALLES, Jürgen, 97723 Oberthulba (DE); RÜFFER, Andreas, 97708 Bad Bocklet (DE); BÜTTNER, Christian, 96215 Lichtenfels (DE); WESTHÄUSER, Florian, 96476 Bad Rodach-Roßfeld (DE)
(74) Vertreter: Paul & Albrecht Patentanwälte PartG mbB

(56) Entgegenhaltungen:
- WO-A1-2020/111959
- US-A- 4 368 733
- US-A1- 2001 044 953
- US-A1- 2016 051 234
- US-A1- 2016 051 445

## Beschreibung

Die vorliegende Erfindung betrifft eine Vorrichtung zum Sammeln einer Stuhlprobe eines Lebewesens, insbesondere eines Menschen, mit einem hohlförmigen Grundkörper, der eine Umfangswand aufweist und an seiner oberen Stirnseite und unteren Stirnseite offen ausgebildet ist, und einem an der Oberseite offenen Sammelbeutel, der einen Aufnahmeraum für eine Stuhlprobe bildet und derart an dem Grundkörper befestigt ist, dass eine aus dem Grundkörper untenseitig austretende Stuhlprobe in dem Sammelbeutel aufgenommen werden kann, wobei im unteren Endbereich des Sammelbeutels ein Filterraum ausgebildet ist, der durch ein blattförmiges Filterelement von dem Aufnahmeraum getrennt ist.

Im menschlichen Darm leben viele Billionen Bakterien. Mehrere Hundert verschiedene Spezies besiedeln insbesondere den Dickdarm und bilden das sogenannte Mikrobiom. Diese Bakterien sind nicht nur für das menschliche Immunsystem relevant, sondern auch für die Verdauung von Ballaststoffen verantwortlich. Sie produzieren Enzyme, welche diese für uns sonst unverwertbaren Nahrungsbestandteile aufspalten. Außerdem wehren sie Krankheitserreger ab und produzieren Vitamine.

Es ist bekannt, dass die Zusammensetzung des Mikrobioms von vielen unterschiedlichen Faktoren, unter anderem auch von der Lebensweise jedes einzelnen Menschen abhängt. Verschiebungen und Ungleichgewicht in der Zusammensetzung der Darmflora können Allergien, die Neigung zur Fettsucht und andere Krankheiten verursachen. Vor diesem Hintergrund ist eine gesunde, ausgewogene Darmflora wichtig für die Gesundheit eines jeden Menschen.

Insofern erfahren der Darm und seine Bakterienflora in den letzten Jahren ein immer größeres Interesse und zwar insbesondere im Rahmen der Beseitigung chronischer Darmentzündungen. Bei einem Befall des Darms mit Bakterieninfektionen, beispielsweise Entzündungen der Darmwand, die von den Giften des Bakteriums Clostridioides difficile herrühren, erfolgt die Behandlung regelmäßig mit Antibiotika. Ein Problem besteht jedoch darin, dass die eingesetzten Antibiotika keinen Unterschied machen zwischen guten und für die Gesundheit unverzichtbaren Bakterien im Darm und denen, durch welche die zu behandelnde Entzündung verursacht wurde. Die Folge ist ein allgemeines Bakteriensterben im Darm der Patienten, und nicht selten breiten sich auf den freien Plätzen der abgestorbenen guten Bakterien die schädlichen Clostridien aus. Häufig folgt eine Entzündung des Darms, bis hin zur sogenannten pseudomembranösen Kolitis, mit einhergehenden hartnäckigen Durchfällen. Nach einer längeren Behandlung mit Antibiotika wird deshalb regelmäßig ein Neuaufbau der Darmflora medikamentös und/oder mit probiotischen Lebensmitteln begleitet.

Eine seit einigen Jahren eingesetzte alternative Behandlung von symptomatischen Infektionen mit Clostridioides difficile, die seit Mitte 2016 auch in Deutschland zugelassen ist, besteht in einer Stuhltransplantation, bei welcher Stuhl von gesunden Spendern in den Darm von Kranken appliziert wird. Dabei wird die Darmflora von einer auf eine andere Person übertragen, was mit unterschiedlichen Begriffen bezeichnet wird. In der englischen Sprache wird meistens der Begriff "Fecal Microbiota Transplantation" verwendet.

Ferner existieren neuere Bestrebungen, wonach eine Eigenstuhltransplantation durchgeführt wird. Das bedeutet, dass insbesondere vor einer Behandlung mit Antibiotika einem Patienten Stuhlproben entnommen werden, die dann längerfristig aufbewahrt werden, um die darin enthaltenen Mikroorganismen nach einer Antibiotikabehandlung in den Darm des Patienten wiedereinzusetzen. Vor dem Hintergrund dieser Therapiemöglichkeiten besteht die Notwendigkeit, eine Stuhlprobe eines Menschen auf einfache und hygienische Weise zu entnehmen und aufzubewahren. Dazu sind Vorrichtungen der eingangs genannten Art beispielsweise aus der WO 2020/111959 A1 vorbekannt. Dieses Dokument wird als nächstliegender Stand der Technik gesehen. Weitere Vorrichtungen, die dem Sammeln vom Stuhlproben dienen, sind aus der US 2001/044953 A1, der US 2016/0051445 A1, der US 2016/0051234 A1 und der US 4,368,733 B vorbekannt.

Vor diesem Hintergrund liegt die Aufgabe der vorliegenden Erfindung darin, eine Vorrichtung bereitzustellen, mit welcher der Stuhl eines Spenders für eine Transplantation auf einfache Weise gewonnen werden kann.

Diese Aufgabe ist gelöst bei einer Vorrichtung der eingangs genannten Art dadurch, dass das Filterelement aus einem quadratischen, einmal über seine Diagonale gefalteten Filterblatt besteht, wobei die offenen, übereinanderliegenden Kanten mit einer unteren Ecke des Sammelbeutels verschweißt sind, sodass der Filterraum in einer Seitenansicht eine im Wesentlichen dreieckige Grundform aufweist.

Der Erfindung liegt die Überlegung zugrunde, dass flüssige Bestandteile der Stuhlprobe das Filterelement durchdringen können und sich im Filterraum ansammeln können. Im Sammelbeutel kann ferner eine Aufbereitung der Stuhlprobe stattfinden. Dazu können Flüssigkeiten hinzugefügt werden, durch welche bestimmte Komponenten, insbesondere die in der Stuhlprobe enthaltenen Mikroorganismen, herausgelöst werden. Diese können dann das Filterelement durchdringen und in den Filterraum eintreten, so dass die Mikroorganismen dem Filterraum entnommen und einem Patienten eingesetzt werden können. Die Vorrichtung kann insbesondere auf einer Toilette unter dem Gesäß des Spenders platziert werden kann, sodass austretender Stuhl in einem Sammelbeutel aufgefangen werden kann. Ein Grundkörper mit einer gewissen Formstabilität bildet dabei eine Durchgangsöffnung zwischen einer oberen Stirnseite und einer unteren Stirnseite, sodass die Stuhlprobe schwerkraftbedingt durch den Grundkörper hindurch in den Sammelbeutel fällt. Die Vorrichtung ermöglicht damit ein hygienisches Entnehmen einer Stuhlprobe eines Spenders.

In weiterer Ausgestaltung kann der Grundkörper aus einem insbesondere einteiligen Zuschnitt aus Karton, Pappe oder dergleichen gefaltet oder faltbar sein. Eine solche Ausgestaltung des Grundkörpers aus Karton oder Pappe führt zu einer entsprechenden Formstabilität, sodass ein Verformen des Grundkörpers, insbesondere wenn er unter dem Gesäß eines Spenders positioniert ist, weitgehend ausgeschlossen ist.

In weiterer Ausgestaltung kann der Abstand zwischen der oberen Stirnseite und der unteren Stirnseite des Grundkörpers mindestens 5 cm, insbesondere mindestens 8 cm, und/oder höchstens 28 cm, insbesondere höchstens 20 cm, bevorzugt 12 cm betragen. Eine derartige Streckung des Grundkörpers hat sich als vorteilhaft herausgestellt, da die entsprechende Vorrichtung ohne weiteres in einer üblichen Toilette positioniert werden kann.

Der Grundkörper kann einen rechteckigen oder einen rautenförmigen Querschnitt, insbesondere einen quadratischen Querschnitt aufweisen, sodass die Umfangswand umlaufend vier insbesondere ebene Seitenwandungen umfasst. Dadurch wird eine ausreichend hohe Formstabilität erreicht. Konkret kann der Grundkörper einen rautenförmigen oder quadratischen Querschnitt aufweisen, wobei die Kantenlänge mindestens 4 cm, insbesondere mindestens 6 cm, und/oder höchstens 15 cm, insbesondere höchstens 12 cm, bevorzugt 8 cm beträgt. Ein entsprechender Querschnitt des Grundkörpers ist zum einen hinreichend groß, dass eine Stuhlprobe den Grundkörper passieren kann, andererseits passt ein solcher Grundkörper ohne Probleme in eine handelsübliche Toilettenschüssel.

In bevorzugter Ausgestaltung kann der Grundkörper Befestigungsmittel zum Anbringen an einem Gegenstand, insbesondere an einer Toilette aufweisen. Diese Ausgestaltung liegt die Überlegung zugrunde, dass der Spender beim Ausscheiden einer Stuhlprobe die erfindungsgemäße Vorrichtung nicht selbst halten muss, sondern diese zuvor an einer Toilette befestigt wird.

Konkret können die Befestigungsmittel zwei Befestigungslaschen umfassen, welche an zwei einander gegenüberliegenden Seiten der Umfangswand von der oberen Stirnseite des Grundkörpers nach außen abragen. Entsprechend ist vorgesehen, dass die Befestigungslaschen zwischen dem Rand einer Toilettenschüssel und der Toilettenbrille befestigt werden können. Konkret können die Befestigungslaschen eine Länge von mindestens 10 cm, insbesondere von 15 cm, und/oder von höchstens 30 cm, insbesondere von höchstens 25 cm, bevorzugt von 18 cm aufweisen. Befestigungslaschen mit einer derartigen Länge, die von einander gegenüberliegenden Seiten der Umfangswand seitlich abragen, sind geeignet, bei üblichen Toiletten zwischen dem Rand einer Toilettenschüssel und der Toilettenbrille positioniert zu werden.

Zum besseren Fixieren können auf der Außenseite der Befestigungslaschen Klebemittel insbesondere in der Form eines Klebestreifens aufgebracht sein, um die Befestigungslaschen an einem Gegenstand, insbesondere am Rand einer Toilette befestigen zu können. Mit einer solchen Ausgestaltung können die Befestigungslaschen nicht nur kraftschlüssig zwischen dem oberen Rand einer Toilettenschüssel und der Toilettenbrille eingeklemmt werden, sondern zusätzlich durch entsprechende Klebemittel stoffschlüssig am Rand einer Toilettenschüssel fixiert werden. Dadurch wird das Risiko eines Verrutschens oder Lösens erheblich verringert. Die Klebestreifen können mit einer Schutzfolie versehen sein, die unmittelbar vor dem Gebrauch der Vorrichtung abgezogen werden kann.

In konkreter Ausgestaltung können die Befestigungslaschen schwenkbar an der oberen Kante zwei einander gegenüberliegender Seitenwandungen angeordnet sein, wobei die Befestigungslaschen insbesondere über eine Falzlinie mit der jeweils angrenzenden Seitenwandung verbunden sind. Das bedeutet, dass die Befestigungslaschen Bestandteil eines einteiligen Zuschnitts aus Karton, Pappe oder dergleichen sein können, aus welchen der Grundkörper gefaltet ist.

Der Sammelbeutel kann derart am Grundkörper befestigt sein, dass die Oberseite den unteren Endbereich des Grundkörpers außenseitig umgibt. Dadurch wird sichergestellt, dass eine Stuhlprobe, welche schwerkraftbedingt den Grundkörper untenseitig verlässt, sicher im Sammelbeutel aufgefangen wird.

An der Oberseite des Sammelbeutels können zwei Haltelaschen angeordnet sein, insbesondere angeschweißt sein, die mit ihren freien Enden an dem Grundkörper, insbesondere an zwei einander gegenüberliegenden Seitenwandungen des Grundkörpers, bevorzugt mit einer Metallklammer befestigt sind. Eine solche Befestigung über zwei Haltelaschen ermöglicht ein einfaches Anbringen des Sammelbeutels an dem Grundkörper, da beispielsweise mit einem Tacker die Haltelaschen nur von außen an dem Grundkörper befestigt werden müssen.

Um den Sammelbeutel durch Abreißen von dem Grundkörper trennen zu können, sind die Haltelaschen bevorzugt mit einer Perforation versehen. Dadurch kann nach der Probenentnahme der Sammelbeutel auf einfache Weise vom Grundkörper entfernt werden, um die Stuhlprobe, welche sich im Sammelbeutel befindet, weiter zu lagern oder aufzubereiten.

Erfindungsgemäß ist das Filterelement blattförmig ausgebildet. Erfindungsgemäß besteht das Filterelement aus einem quadratischen, einmal über seine Diagonale gefalteten Filterblatt, wobei die offenen, übereinanderliegenden Kanten mit einer unteren Ecke des Sammelbeutels verschweißt sind, sodass der Filterraum in einer Seitenansicht eine im wesentlichen dreieckige Grundform aufweist. Mit anderen Worten ist in einer unteren Ecke des Sammelbeutels ein Filterraum ausgebildet, der durch ein Filterblatt, welches einmal über seine Diagonale gefaltet ist, von dem Aufnahmeraum des Sammelbeutels getrennt ist. Der Filterraum weist dabei eine im Wesentlichen dreieckige Grundform auf, wenn man den Sammelbeutel von der Seite betrachtet.

Das Filterelement kann eine Maschenweite von mindestens 5 µm, insbesondere von mindestens 25 µm, bevorzugt von mindestens 40 µm, und/oder von höchstens 150 µm, insbesondere von höchstens 100 µm, bevorzugt von höchstens 70 µm, besonders bevorzugt eine Maschenweite von 50 µm aufweisen. Derartige Maschenweiten haben sich als vortheilhaft herausgestellt, da sie zuverlässig in einer Flüssigkeit gelöste oder von einer Flüssigkeit getragene Mikroorganismen durchlassen, größere Stücke der Stuhlprobe jedoch zuverlässig zurückhalten.

Das Filterelement kann zumindest im Wesentlichen aus Polyethylenterephthalat (PET) und/oder aus Polyamid (PA) bestehen. Solche Materialien sind chemisch hinreichend inert, um eine Veränderung von Mikroorganismen, welche das Filterelement passieren, zu verhindern.

Untenseitig kann am Sammelbeutel ein Ventil angebracht sein, welches mit dem Filterraum fluidisch verbunden ist, um den Filterraum entleeren oder mit einer Flüssigkeit befüllen zu können. Bevorzugt ist das Ventil als Injektionsport ausgebildet. Dementsprechend kann der Filterraum, welchem sich insbesondere eine Flüssigkeit mit den in der Stuhlprobe enthaltenen Mikroorganismen befindet, auf einfache Weise entleert werden. Ein Injektionsport ist dadurch gekennzeichnet, dass er eine Trennbarriere bildet zwischen dem Filterraum und der Umgebung, welche von einer Spritze durchstochen werden kann. Damit kann in an sich bekannter Weise die Flüssigkeit aus dem Filterraum entnommen werden oder in den Filterraum eingebracht werden.

In weiterer Ausgestaltung kann die erfindungsgemäße Vorrichtung dadurch gekennzeichnet sein, dass ferner ein Innenbeutel vorgesehen ist, welcher die Umfangswand des Grundkörpers innenseitig umlaufend auskleidet. Dieser Innenbeutel hat die Funktion, dass die Stuhlprobe nicht mit der Umfangswand in Kontakt kommen kann. Vielmehr führt der Innenbeutel die Stuhlprobe, welche schwerkraftbedingt in Richtung des Sammelbeutels fällt. Dementsprechend ist der Innenbeutel nach oben und unten offen ausgebildet.

Bevorzugt erstreckt sich der Innenbeutel über die gesamte Strecke des Grundkörpers zwischen der oberen Stirnseite und der unteren Stirnseite. Ferner kann der Innenbeutel an seiner Oberseite einen Ringbund aufweisen, welcher die obere Stirnseite des Grundkörpers überdeckt. Ein solcher Ringbund verhindert, dass Bestandteile einer insbesondere dünnflüssigeren Stuhlprobe außen an dem Grundkörper entlang strömen. Der Ringbund wirkt hier praktisch wie ein Trichter, welcher die Stuhlprobe zum Innenraum des Grundkörpers führt. Der Innenbeutel kann dabei auf verschiedene Arten an dem Grundkörper befestigt sein. Beispielsweise kann der Ringbund über Metallklammern an der Umfangswand oder an Befestigungslaschen fixiert sein. Möglich ist auch, dass entsprechende Schlaufen an den Innenbeutel, insbesondere an dem Ringbund, angeklebt sind, um den Innenbeutel an dem Grundkörper zu fixieren.

In weiterer Ausgestaltung kann der Innenbeutel untenseitig aus dem Grundkörper vorragen. Dementsprechend ist ausgeschlossen, dass die Stuhlprobe beim untenseitigen Verlassen des Innenbeutels mit dem Grundkörper in Kontakt kommt. Bevorzugt ist der Innenbeutel insbesondere an zwei einander gegenüberliegenden Stellen mit dem Sammelbeutel verbunden, insbesondere verschweißt. Dadurch wird ein stabiler Aufbau des Innenbeutels und des Sammelbeutels realisiert. Ferner kann durch den Zwischenraum zwischen dem Innenbeutel und dem Sammelbeutel von außen Flüssigkeit hinzugefügt werden, wenn die Stuhlprobe sich bereits im Sammelbeutel befindet. Ein Verschweißen der beiden Beutel miteinander stellt dabei eine einfache Fertigungsmöglichkeit dar. In den Verbindungsstellen kann ferner eine Sollbruchstelle oder Sollrisslinie vorgesehen sein, beispielsweise eine Perforation welche aufgetrennt werden kann, wenn der Sammelbeutel vom Innenbeutel bzw. dem Grundkörper getrennt werden soll.

In konkreter Ausgestaltung können der Sammelbeutel und/oder die Haltelasche und/oder der Innenbeutel aus einer Folie aus Polyethylen (PE) mit einer Dicke von mindestens 50 µm, insbesondere mindestens 100 µm, und/oder höchstens 300 µm, insbesondere höchstens 250 µm bestehen. Ein derartiger Dickebereich ist vorteilhaft, da derartige Beutel einerseits eine hinreichende Stabilität haben, andererseits jedoch hinreichend flexibel sind.

Für die weitere Ausgestaltung der Erfindung wird auf die Unteransprüche sowie auf das nachstehend beschriebene Ausführungsbeispiel unter Bezugnahme auf die Zeichnung verwiesen. In der Zeichnung zeigen:
- Figur 1: eine Vorrichtung zum Sammeln einer Stuhlprobe eines Menschen gemäß der vorliegenden Erfindung in einer perspektivischen Darstellung in einem zusammengefalteten Zustand;
- Figur 2: die Vorrichtung aus Figur 1 in einer schematischen Längsschnittansicht;
- Figur 3: den Sammelbeutel der Vorrichtung der Figuren 1 und 2 in einer Seitenansicht;
- Figur 4: das Filterelement der Vorrichtung aus Figur 1 und Figur 2;
- Figur 5: ein einteiliger Zuschnitt des Grundkörpers der Vorrichtung aus Figur 1 und Figur 2.

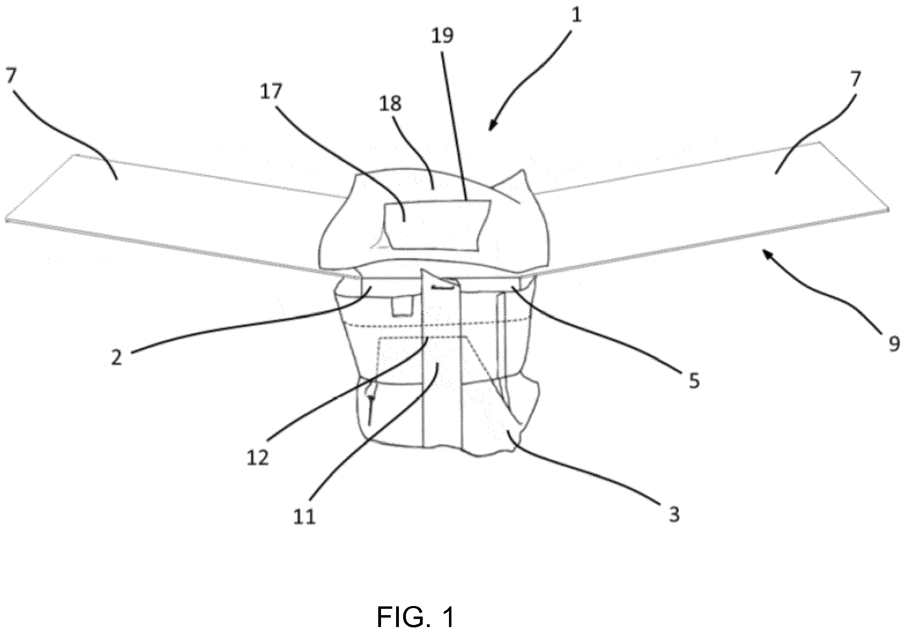

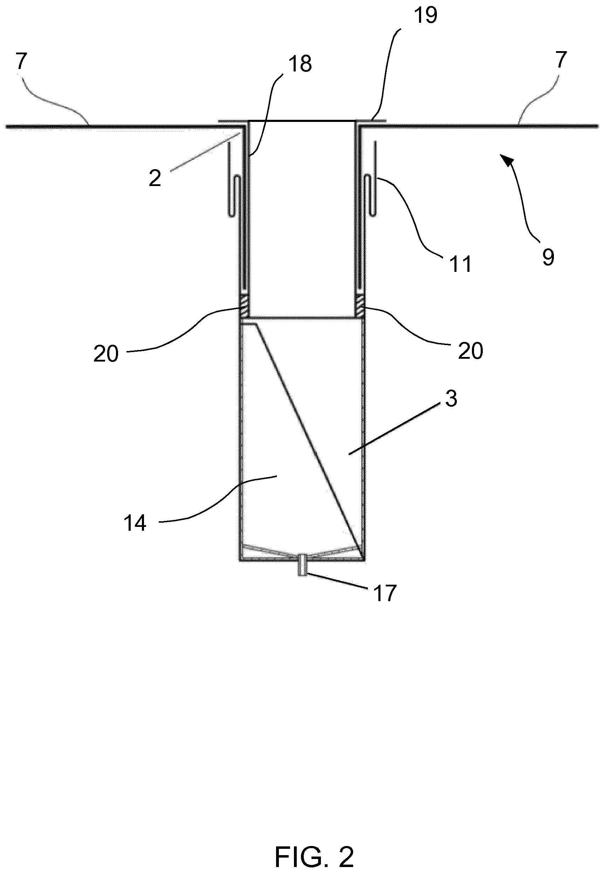

Die Figur 1 und die Figur 2 zeigen eine Vorrichtung 1 zum Sammeln einer Stuhlprobe eines Menschen, welche einen hohlförmigen Grundkörper 2 und einen Sammelbeutel 3, der einen Aufnahmeraum für die Stuhlprobe bildet, aufweist.

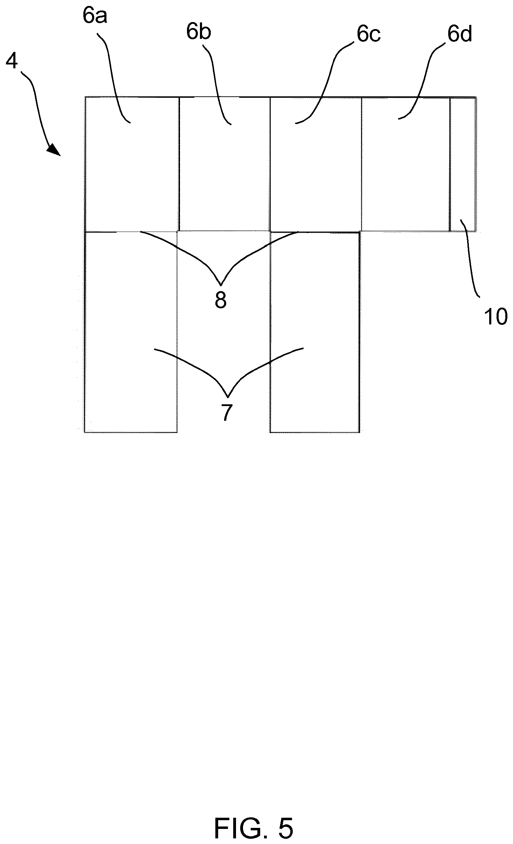

Der Grundköper 2 ist aus einem einteiligen Zuschnitt 4 aus Karton gefaltet. Der Zuschnitt 4 ist im Detail in Figur 5 dargestellt.

Der Grundkörper 2 weist eine Umfangswand 5 auf, die vier rechteckige Seitenwandungen 6a, 6b, 6c, 6d umfasst. An einer oberen Stirnseite und an einer unteren Stirnseite ist der Grundkörper 2 offen ausgebildet und besitzt einen rechteckigen Querschnitt, wobei die Kantenlänge vorliegend 81 mm beträgt.

Ferner weist der Grundkörper 2 Befestigungsmittel zum Anbringen an einer Toilette, insbesondere zwischen dem oberen Rand einer Toilettenschüssel und einer Toilettenbrille auf. Die Befestigungsmittel umfassen zwei Befestigungslaschen 7, welche an einander gegenüberliegenden Seitenwandungen 6a, 6c angeordnet sind. In der Figur 5 ist erkennbar, dass die beiden Befestigungslaschen 7 in dem Zuschnitt 4 jeweils über Falzlinie 8 mit den Seitenwandungen 6a, 6c verbunden sind. Die Befestigungslaschen 7 mit einer Länge von 18 cm sind ferner mit einem Klebestreifen 9 versehen, um die Befestigungslaschen 7 am Rand einer Toilette befestigen zu können.

In der Figur 5 ist erkennbar, dass die Seitenwandungen 6a, 6b, 6c und 6d über Falzlinien 8 miteinander verbunden sind. An die Seitenwandung 6d schließt sich in dem Zuschnitt 4 außenseitig über eine Falzlinie 8 eine Montagelasche 10 an, die beim Zusammenfalten zum Grundkörper mit der gegenüberliegenden Seitenwandung 6a verbunden, insbesondere verklebt wird.

Der Sammelbeutel 3 bildet einen Aufnahmeraum für eine Stuhlprobe und ist derart an dem Grundkörper 2 befestigt, dass eine aus dem Grundkörper 2 untenseitig austretende Stuhlprobe in dem Sammelbeutel 3 aufgenommen werden kann. Dazu umgibt die Oberseite des Sammelbeutels 3 den unteren Endbereich des Grundkörpers 2 außenseitig.

An der Oberseite des Sammelbeutels 3 sind zwei Haltelaschen 11 angeschweißt, die mit ihren freien Enden an dem Grundkörper 2 zwei einander gegenüberliegenden Seitenwandungen 6b, 6d mittels einer Metallklammer befestigt sind. Die Haltelaschen 11 sind dabei mit einer Perforation 12 versehen, um den Sammelbeutel 3 durch Abreißen von dem Grundkörper 2 trennen zu können.

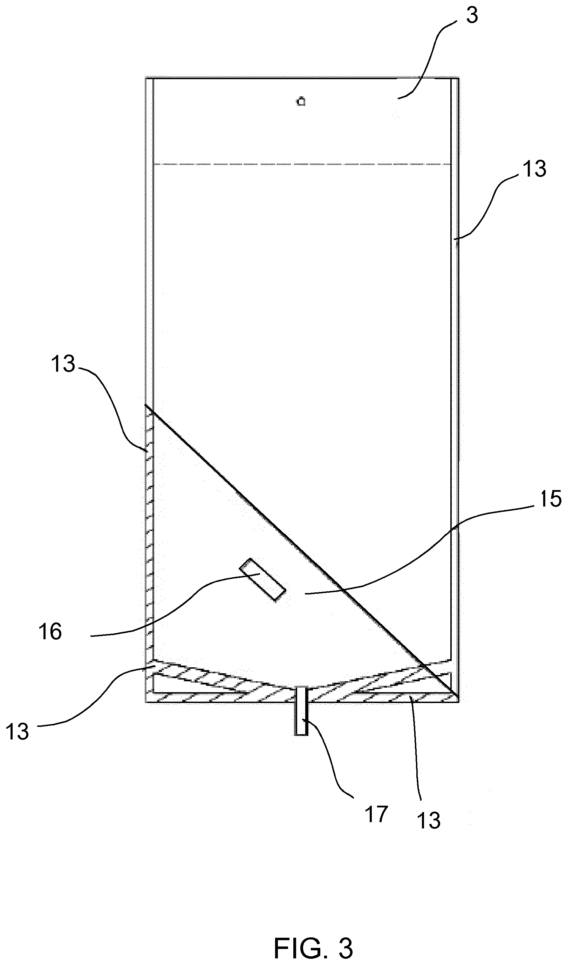

Die konkrete Ausgestaltung des Sammelbeutels ist in der Figur 3 im Detail dargestellt. Der Sammelbeutel 3 wird hergestellt, indem zwei Folien aus Polyethylen mit einer Dicke von jeweils 200 µm aufeinandergelegt werden und entlang einer Schweißlinie 13 miteinander verschweißt werden. Der untere Endbereich des Sammelbeutels 3 ist dabei trichterförmig verschweißt, das heißt die Schweißlinie 13 ist in diesem Bereich schräg ausgebildet.

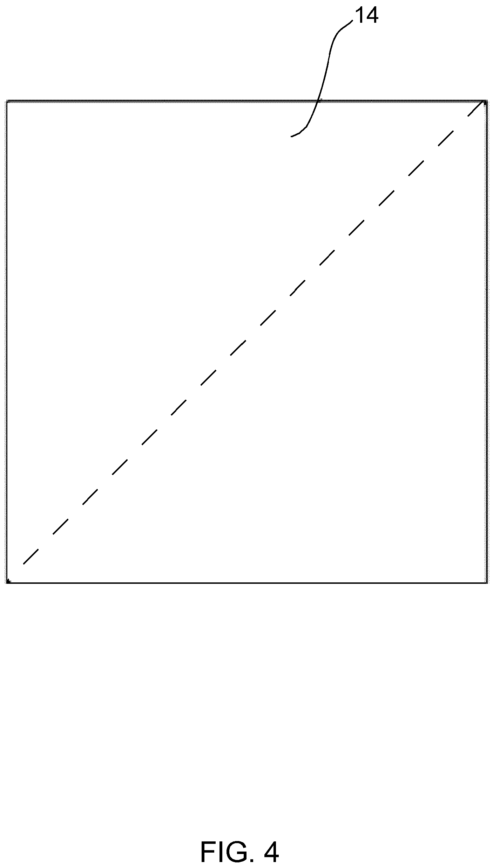

Im unteren Endbereich des Sammelbeutels 3 ist ein Filterraum 14 ausgebildet, der durch ein Filterelement 15 von dem Aufnahmeraum getrennt ist. Das Filterelement 15, welches in der Figur 4 im Detail dargestellt ist, ist blattförmig ausgebildet und besteht aus einem quadratischen Filterblatt, welches einmal über seine Diagonale gefaltet ist, wie es in der Figur 4 anhand der gestrichelten Linie dargestellt ist. Die offenen, übereinanderliegenden Kanten sind dabei mit einer unteren Ecke des Sammelbeutels 3 verschweißt. Somit ergibt sich wie in der Figur 3 erkennbar ist einen Filterraum 14 mit einer im Wesentlichen dreieckigen Grundform. Zusätzlich sind an den beiden einander gegenüberliegenden Folien des Sammelbeutels 3 jeweils zwei Schweißpunkte 16 vorgesehen, an welchem das Filterelement 15 mit den Folien des Sammelbeutels 3 verschweißt ist. Dadurch wird erreicht, dass sich, wenn sich eine Stuhlprobe in dem Sammelbeutel 3 befindet, die Folien des Sammelbeutels voneinander trennen und somit der Filterraum 14 eine volumenmäßige Ausdehnung erreicht.

Untenseitig am Sammelbeutel 3 ist ein Entnahmeventil 17 angebracht. Dieses ist mit dem Filterraum 14 fluidisch verbunden, um den Filterraum 14 entleeren zu können. Konkret ist das Entnahmeventil 17 als Injektionsport ausgebildet.

Die Vorrichtung 1 umfasst ferner einen Innenbeutel 18, welcher die Umfangswand 5 des Grundkörpers 2 innenseitig umlaufend auskleidet. Dadurch wird verhindert, dass eine Stuhlprobe in direkten Kontakt mit dem Grundkörper 2 aus Pappe kommt. Der oben und unten offene Innenbeutel 18 erstreckt sich dabei zwischen der oberen Stirnseite und der unteren Stirnseite des Grundkörpers 2 und weist an seiner Oberseite einen Ringbund 19 auf, welcher die obere Stirnseite des Grundkörpers 2 überdeckt.

Untenseitig ragt der Innenbeutel 18 aus dem Grundkörper vor, das heißt bis in den Sammelbeutel 3 hinein. An zwei einander gegenüberliegenden Verbindungsstellen 20 ist der Innenbeutel 18 mit dem Sammelbeutel 3 verschweißt. Dadurch ist sichergestellt, dass eine Stuhlprobe den Grundkörper 2 den Innenbeutel 18 passieren kann und in den Sammelbeutel 3 gelangt.

Die Vorrichtung 1 kann damit zur Entnahme einer Stuhlprobe mit den Befestigungslaschen 7 zwischen dem oberen Rand einer Toilettenschüssel und der Toilettenbrille positioniert werden. Beim Ausscheiden einer Stuhlprobe fällt diese durch den Innenbeutel 18, welcher die Innenwand des Grundkörpers 2 auskleidet, in den Sammelbeutel 3. Dieser kann mittels der Perforation an den Haltelaschen 11 von dem Grundkörper 2 getrennt werden.

Die Stuhlprobe kann durch Hinzugabe von Flüssigkeit aufbereitet werden. Das bedeutet, dass beispielsweise durch das Ventil 17 Flüssigkeit in den Filterraum 14 eingebracht werden kann. Mikroorganismen, welche in der Stuhlprobe enthalten sind, können das Filterelement 15 passieren und sich im Filterraum 14 sammeln. Durch das Entnahmeventil 17 kann auf einfache Weise die so im Filterraum 14 gesammelte Flüssigkeit, welche Mikroorganismen enthält, bedarfsweise entnommen werden.

### Bezugszeichenliste

- 1: Vorrichtung
- 2: Grundkörper
- 3: Sammelbeutel
- 4: Zuschnitt
- 5: Umfangswand
- 6a, 6b, 6c. 6d: Seitenwandung
- 7: Befestigungslasche
- 8: Falzlinie
- 9: Klebestreifen
- 10: Montagelasche
- 11: Haltelasche
- 12: Perforation
- 13: Schweißlinie
- 14: Filterraum
- 15: Filterelement
- 16: Schweißpunkt
- 17: Entnahmeventil
- 18: Innenbeutel
- 19: Ringbund
- 20: Verbindungsstelle

## Patentansprüche

1. Vorrichtung (1) zum Sammeln einer Stuhlprobe eines Lebewesens, insbesondere eines Menschen, mit
einem hohlförmigen Grundkörper (2), der eine Umfangswand (5) aufweist und an seiner oberen Stirnseite und unteren Stirnseite offen ausgebildet ist, und
einem an seiner Oberseite offenen Sammelbeutel (3), der einen Aufnahmeraum für eine Stuhlprobe bildet und derart an dem Grundkörper (2) befestigt ist, dass eine aus dem Grundkörper (2) untenseitig austretende Stuhlprobe in dem Sammelbeutel (3) aufgenommen werden kann,
wobei im unteren Endbereich des Sammelbeutels (3) ein Filterraum (14) ausgebildet ist, der durch ein blattförmiges Filterelement (15) von dem Aufnahmeraum getrennt ist, **dadurch gekennzeichnet, dass** das Filterelement (15) aus einem quadratischen, einmal über seine Diagonale gefalteten Filterblatt besteht, wobei die offenen, übereinanderliegenden Kanten mit einer unteren Ecke des Sammelbeutels (3) verschweißt sind, sodass der Filterraum (14) in einer Seitenansicht eine im Wesentlichen dreieckige Grundform aufweist.

2. Vorrichtung (1) nach Anspruch 1, **dadurch gekennzeichnet, dass** der Grundkörper (2) aus einem insbesondere einteiligen Zuschnitt (4) aus Karton, Pappe oder dergleichen gefaltet oder faltbar ist.

3. Vorrichtung (1) nach einem der vorherigen Ansprüche, **dadurch gekennzeichnet, dass** der Grundkörper (2) einen rechteckigen oder einen rautenförmigen Querschnitt, insbesondere einen quadratischen Querschnitt aufweist, so dass die Umfangswand (5) umlaufend vier insbesondere ebene Seitenwandungen (6a, 6b, 6c, 6d) umfasst.

4. Vorrichtung (1) nach Anspruch 3, **dadurch gekennzeichnet, dass** der Grundkörper (2) einen rautenförmigen, insbesondere einen quadratischen Querschnitt aufweist, wobei die Kantenlänge mindestens 4 cm, insbesondere mindestens 6 cm, und/oder höchstens 15 cm, insbesondere höchstens 12 cm, bevorzugt 8 cm beträgt.

5. Vorrichtung (1) nach einem der vorherigen Ansprüche, **dadurch gekennzeichnet, dass** der Grundkörper (2) Befestigungsmittel zum Anbringen an einem Gegenstand, insbesondere an einer Toilette aufweist.

6. Vorrichtung (1) nach einem der vorherigen Ansprüche, **dadurch gekennzeichnet, dass** der Sammelbeutel (3) derart am Grundkörper (2) befestigt ist, dass die Oberseite den unteren Endbereich des Grundkörpers (2) außenseitig umgibt.

7. Vorrichtung (1) nach Anspruch 6, **dadurch gekennzeichnet, dass** an der Oberseite des Sammelbeutels (3) zwei Haltelaschen (11) angeordnet, insbesondere angeschweißt sind, die mit ihren freien Enden an dem Grundkörper (2), insbesondere an zwei einander gegenüberliegenden Seitenwandungen des Grundkörpers (2), bevorzugt mit einer Metallklammer befestigt sind.

8. Vorrichtung (1) nach Anspruch 7, **dadurch gekennzeichnet, dass** die Haltelaschen (11) mit einer Perforation (12) versehen sind, um den Sammelbeutel (3) durch Abreißen von dem Grundkörper (2) trennen zu können.

9. Vorrichtung (1) nach einem der vorherigen Ansprüche, **dadurch gekennzeichnet, dass** das Filterelement (15) eine Maschenweite von mindestens 5 µm, insbesondere von mindestens 25 µm, bevorzugt von mindestens 40 µm, und/oder von höchstens 150 µm, insbesondere von höchstens 100 µm, bevorzugt von höchstens 70 µm, besonders bevorzugt eine Maschenweite von genau 50 µm aufweist.

10. Vorrichtung (1) nach einem der vorherigen Ansprüche, **dadurch gekennzeichnet, dass** das Filterelement (15) zumindest im Wesentlichen aus Polyethylenterephthalat (PET) und/oder Polyamid (PA) besteht, und/oder dass untenseitig am Sammelbeutel (3) ein Ventil (17) angebracht ist und mit dem Filterraum (14) fluidisch verbunden ist, um den Filterraum (14) entleeren oder mit einer Flüssigkeit befüllen zu können.

11. Vorrichtung (1) nach einem der vorherigen Ansprüche, **dadurch gekennzeichnet, dass** ferner ein Innenbeutel (18) vorgesehen ist, welcher die Umfangswand (5) des Grundkörpers (2) innenseitig umlaufend auskleidet.

12. Vorrichtung (1) nach Anspruch 11, **dadurch gekennzeichnet, dass** sich der Innenbeutel (18) über die gesamte Strecke des Grundkörpers (2) zwischen der oberen Stirnseite und der unteren Stirnseite erstreckt.

13. Vorrichtung (1) nach einem der vorherigen Ansprüche, **dadurch gekennzeichnet, dass** der Sammelbeutel (3) und/oder der Innenbeutel (18) aus einer Folie aus Polyethylen mit einer Dicke von mindestens 50 µm, insbesondere mindestens 100 µm, und/oder höchstens 300 µm, insbesondere höchstens 250 µm besteht.

## Claims

1. A device (1) for collecting a stool sample from a living being, in particular a human, comprising
a hollow base body (2) having a peripheral wall (5) and open at its upper end face and lower end face, and
a collection bag (3) open at its top, which forms a receiving chamber for a stool sample and is attached to the base body (2) in such a way that a stool sample exiting the base body (2) from the bottom can be collected in the collection bag (3),
wherein a filter chamber (14) is formed in the lower end region of the collection bag (3), which is separated from the collection chamber by a sheet-like filter element (15), **characterized in that** the filter element (15) consists of a square filter sheet folded once along its diagonal, wherein the open, overlapping edges are heat-sealed to a lower corner of the collection bag (3), such that the filter chamber (14) has a substantially triangular basic shape when viewed from the side.

2. Device (1) according to claim 1, **characterized in that** the main body (2) is folded or foldable from a blank (4)-in particular a one-piece blank-made of cardboard, paperboard, or the like.

3. A device (1) according to any of the preceding claims, **characterized in that** the base body (2) has a rectangular or diamond-shaped cross-section, in particular a square cross-section, such that the peripheral wall (5) comprises four, in particularr flat, side walls (6a, 6b, 6c, 6d) extending around its perimeter.

4. Device (1) according to claim 3, **characterized in that** the base body (2) has a diamond-shaped, in particular a square, cross-section, wherein the edge length is at least 4 cm, in particular at least 6 cm, and/or at most 15 cm, in particular at most 12 cm, preferably 8 cm.

5. A device (1) according to one of the preceding claims, **characterized in that** the base body (2) comprises fastening means for attaching it to an object, in particular to a toilet.

6. Device (1) according to one of the preceding claims, **characterized in that** the collection bag (3) is attached to the base body (2) such that the top side surrounds the lower end region of the base body (2) on the outside.

7. A device (1) according to claim 6, **characterized in that** two retaining tabs (11) are arranged on the top side of the collection bag (3)- , in particular, welded-which are fastened with their free ends to the base body (2), in particular to two opposing side walls of the base body (2), preferably with a metal clip.

8. Device (1) according to claim 7, **characterized in that** the retaining tabs (11) are provided with a perforation (12) to allow the collection bag (3) to be separated from the base body (2) by tearing it off.

9. A device (1) according to any of the preceding claims, **characterized in that** the filter element (15) has a mesh size of at least 5 µm, in particular at least 25 µm, preferably at least 40 µm, and/or a maximum of 150 µm, in particular a maximum of 100 µm, preferably a maximum of 70 µm, and most preferably a mesh size of exactly 50 µm.

10. A device (1) according to one of the preceding claims, **characterized in that** the filter element (15) consists at least substantially of polyethylene terephthalate (PET) and/or polyamide (PA), and/or
that a valve (17) is attached to the bottom of the collection bag (3) and is fluid-ically connected to the filter chamber (14) so that the filter chamber (14) can be emptied or filled with a liquid.

11. A device (1) according to one of the preceding claims, **characterized in that** an inner bag (18) is further provided, which lines the inner circumference of the peripheral wall (5) of the base body (2).

12. A device (1) according to claim 11, **characterized in that** the inner bag (18) extends over the entire length of the base body (2) between the upper end face and the lower end face.

13. A device (1) according to one of the preceding claims, **characterized in that** the collection bag (3) and/or the inner bag (18) consists of a polyethylene film having a thickness of at least 50 µm, in particular at least 100 µm, and/or at most 300 µm, in particular at most 250 µm.

## Revendications

1. Dispositif (1) destiné à recueillir un échantillon de selles d'un être vivant, en particulier d'un être humain, comprenant
un corps de base creux (2) qui présente une paroi périphérique (5) et qui est ouvert à saface supérieure et saface inférieure, et
un sac de collecte (3) ouvert sur sa face supérieure, qui forme un espace de réception pour un échantillon de selles et qui est fixé au corps de base (2) de telle sorte qu'un échantillon de selles sortant par le bas du corps de base (2) puisse être recueilli dans le sac de collecte (3),
un compartiment filtrant (14) étant formé dans la zone d'extrémité inférieure du sac de collecte (3), lequel est séparé de l'espace de réception par un élément filtrant en forme de feuille (15), **caractérisé en ce que** l'élément filtrant (15) est constitué d'une feuille filtrante carrée, pliée une fois selon sa diagonale, les bords ouverts superposés étant soudés à un coin inférieur du sac de collecte (3), de sorte que l'espace filtrant (14) présente, en vue de profil, une forme de base essentiellement triangulaire.

2. Dispositif (1) selon la revendication 1, **caractérisé en ce que** le corps de base (2) est plié ou pliable à partir d'un flan (4), notamment d'une seule pièce, en carton, en papier cartonné ou similaire.

3. Dispositif (1) selon l'une des revendications précédentes, **caractérisé en ce que** le corps de base (2) présente une section transversale rectangulaire ou en forme de losange, en particulier une section transversale carrée, de sorte que la paroi périphérique (5) comprend sur tout son pourtour quatre parois latérales (6a, 6b, 6c, 6d) notamment planes.

4. Dispositif (1) selon la revendication 3, **caractérisé en ce que** le corps de base (2) présente une section transversale en forme de losange, en particulier une section carrée, la longueur des arêtes étant d'au moins 4 cm, en particulier d'au moins 6 cm, et/ou d'au plus 15 cm, en particulier d'au plus 12 cm, de préférence de 8 cm.

5. Dispositif (1) selon l'une des revendications précédentes, **caractérisé en ce que** le corps de base (2) comporte des moyens de fixation destinés à la fixation sur un objet, en particulier sur des toilettes.

6. Dispositif (1) selon l'une des revendications précédentes, **caractérisé en ce que** le sac collecteur (3) est fixé au corps de base (2) de telle sorte que sa face supérieure entoure la partie d'extrémité inférieure du corps de base (2) par l'extérieur.

7. Dispositif (1) selon la revendication 6, **caractérisé en ce que** deux pattes de retenue (11) sont disposées sur la face supérieure du sac collecteur (3), notamment soudées, et sont fixées par leurs extrémités libres au corps de base (2), notamment à deux parois latérales opposées du corps de base (2), de préférence à l'aide d'une agrafe métallique.

8. Dispositif (1) selon la revendication 7, **caractérisé en ce que** les pattes de fixation (11) sont pourvues d'une perforation (12) permettant de séparer le sac collecteur (3) du corps de base (2) par déchirement.

9. Dispositif (1) selon l'une des revendications précédentes, **caractérisé en ce que** l'élément filtrant (15) présente une ouverture de maille d'au moins 5 µm, en particulier d'au moins 25 µm, de préférence d'au moins 40 µm, et/ou d'au plus 150 µm, en particulier d'au plus 100 µm, de préférence d'au plus 70 µm, et de manière particulièrement préférée, une ouverture de maille de exactement 50 µm.

10. Dispositif (1) selon l'une des revendications précédentes, **caractérisé en ce que** l'élément filtrant (15) est constitué au moins essentiellement de polyéthylène téréphtalate (PET) et/ou de polyamide (PA), et/ou
qu'une valve (17) est montée sur la face inférieure du sac collecteur (3) et est reliée de manière fluidique à la chambre de filtration (14), afin de pouvoir vider la chambre de filtration (14) ou la remplir d'un liquide.

11. Dispositif (1) selon l'une des revendications précédentes, **caractérisé en ce qu'**il est en outre prévu un sac intérieur (18) qui recouvre de manière périphérique la paroi périphérique (5) du corps de base (2) sur sa face intérieure.

12. Dispositif (1) selon la revendication 11, **caractérisé en ce que** la poche intérieure (18) s'étend sur toute la longueur du corps de base (2) entre la face frontale supérieure et la face frontale inférieure.

13. Dispositif (1) selon l'une des revendications précédentes, **caractérisé en ce que** le sac collecteur (3) et/ou le sac intérieur (18) est constitué d'un film de polyéthylène d'une épaisseur d'au moins 50 µm, en particulier d'au moins 100 µm, et/ou d'au plus 300 µm, en particulier d'au plus 250 µm.
